# EUROPEAN PATENT SPECIFICATION

(11) **EP 2 957 272 B1**
(45) Date of publication and mention of the grant of the patent: **11.03.2020**
(21) Application number: 15001432.2
(22) Date of filing: 13.05.2015
(51) Int. Cl.: A61G 13/10, A61G 12/00

(54) **FLOOR-BASED MEDICAL EQUIPMENT SUPPORT SYSTEM**
BODENGESTÜTZTES TRAGESYSTEM FÜR MEDIZINISCHE AUSRÜSTUNG
SYSTÈME BASÉ AU SOL DE SUPPORT D'ÉQUIPEMENT MÉDICAL

(30) Priority: 19.05.2014 DE 102014007357
(43) Date of publication of application: 23.12.2015
(73) Proprietor: The University of Dundee, Dundee, Scotland DD1 4HN (GB)
(72) Inventor: Coleman, Stuart, Dundee Medipark, Dundee DD2 1FD, Scotland (GB); Cuschieri, Alfred, Dundee Medipark, Dundee DD2 1FD, Scotland (GB); Brown, Stuart, Dundee Medipark, Dundee DD2 1FD, Scotland (GB)
(74) Representative: Weidner Stern Jeschke

(56) References cited:
- WO-A1-98/10150
- DE-A1-102005 031 557
- DE-U1-202005 006 552

## Description

The present invention relates to a medical equipment support system.

The execution of medical care in primary and secondary care settings increasingly relies upon medical devices and technological systems to meet the patient's needs. Such devices are invariably electronic or electromechanical, must be connected to a source of power and possibly to fluids and will occupy space around the patient. In order to avoid congestion of the patient environment with equipment, cables, hoses etc. and in order to improve access by the care staff to the patient, it is well-known to accommodate medical equipment on ceiling-mounted structures. In this way equipment, cables and other connections can be suspended above the workspace where they cause less inconvenience and allow the care-giver unimpeded access to the patient.

In DE 10 2007 051 038 A1 an equipment carrier segment is disclosed that comprises a substantially horizontal supply beam for accommodating a medical device and a base-holder to which the supply beam is mounted. The base-holder is premounted on the ceiling of an operating or intensive care room in a hospital by means of several vertical struts, which are fixed to the ceiling. The base-holder is mounted to the building structure, the struts being adjustable in length in order to compensate for variations in the ceiling height. An equipment support system comprises at least two equipment carrier segments.

According to US 6,089,518 a supportive structure is attached to a ceiling of a hospital room for supporting hospital equipment. The supporting structure comprises beams attached to the ceiling and forming a rectangular space. Inside the space there are non-interchangeable gas connectors and an electric box connected to the supply of the hospital. In EP 0 215 212 A2 a supply unit for a medical nursing post is disclosed that comprises a supply beam fixed to the lower ends of columns hanging from the ceiling of a room. The supply beam comprises a longitudinal rail along which a carriage can be moved from which a carrier device for medical devices is suspended. Supply cables and hoses pass from the ceiling through the hollow columns.

The aforementioned mounting, suspension or support systems rely upon structural features of the ceiling of a medical room where the system is installed. Special facilities and a load bearing structure may be most conveniently incorporated into a building from the outset. If such a system is to be installed retrospectively in an existing building, considerable expenses are incurred for the necessary structural changes to the medical room. Moreover, these are permanent changes, which may not be desirable or practical for all rooms. Such a fixed layout reduces the ability to adapt to changing patient care requirements or changing technology. Since technology for patient care is rapidly evolving, adaptability of accommodation to suit new technologies (i.e. "future proofing") is an increasing need. Moreover, with the continued evolution of medical technology hospitals may need to change the configuration and purpose of rooms several times during the lifetime of a primary hospital building.

It is also known to accommodate medical equipment on a stand structure. According to EP 0 293 227 A2, medical optical equipment is supported by a parallel linkage supported pivotally on a swivel stand shaft set upright for swivel motion on a base. However, such a stand structure often lacks sufficient stability, in particular if heavy-weight medical equipment is to be supported.

In DE 10 2005 031 557 A1 a carrier system for medical-optical equipment is disclosed. The medical-optical equipment is provided on a portal-shaped carrier unit comprising a carrier beam on which a reception unit for medical-optical equipment is movably guided for moving in a horizontal direction. The portal-shaped carrier unit is supported on guide rails arranged on both sides of an operating table or on consoles having rollers guided on the floor of an operating theatre, or the operating table is movably mounted on the portal-shaped carrier unit. The carrier system is intended in particular for ophthalmologic operations in which precise positioning of a surgical microscope in a horizontal plane is required. However, the portal-shaped carrier unit impedes access to the patient.

According to WO 98/10150 A1 a mounting system for hospitals includes upright girders close to the walls of a room, other upright or vertical girders mounted at a distance from the first-mentioned girders, the system of vertical girders being interconnected at their upper ends by several horizontal girders. Horizontal smaller girders interconnect the first-mentioned horizontal girders.

In DE 20 2005 006 552 U1 an intensive-care and emergency patient transport system is disclosed having a docking cart which may stand beside an intensive-care bed or be docketed to the bed. An equipment carrier can be moved in a gantry-like manner over the bed.

It is an object of the present invention to provide a medical equipment support system which alleviates the above mentioned problems. In particular, it is an object of the invention to provide a medical equipment support system that is not or is less dependent on dedicated permanent structures of a medical room in which it is placed, for example in an operating theatre, and which provides sufficient stability and in particular permits unimpeded access by medical staff to a patient placed on an operating table.

This object is met by a medical equipment support system according to claim 1. Advantageous embodiments of the present invention are indicated in the dependent claims.

According to the present invention, a medical equipment support system comprises a support structure extending in a substantially horizontal direction for supporting directly or indirectly at least one medical device. In particular, the support structure is an elongated structure extending in a horizontal direction. The medical equipment support system further comprises at least two supports arranged at opposing ends of the support structure for supporting the support structure in an overhead position with respect to medical staff performing a medical procedure, in particular a surgical procedure. The support structure is supported in an overhead position which in particular is sufficiently high for medical staff to stand or pass easily underneath. For example the support structure may be supported at a height of about or more than 2 m above the floor. The at least one medical device may be carried by a holder, the holder being attached to the support structure. The holder is configured for holding one or several medical devices, which may be of one particular kind of medical equipment, or of a wide variety of different kinds of equipment. In particular, the medical device can be suspended from the support structure in a position in which it is readily accessible to medical staff without impeding access to the patient. A medical device suspended from the support structure is in a fixed or in a variable relationship to the support structure, i.e. the medical equipment may be held fixedly in a pre-determined position and orientation, may be adjustable or may be movable with respect to the support structure. The holder may be configured such that a medical device held by the holder can be held or moved into a position near, above or about the same level as the operating table. In particular, the holder on which the device is mountable may be or comprise an adjustable or articulating support arm which in turn is mounted on the support structure in a fixed or in a variable position. In this way adjustment of medical equipment to a wide variety of care settings can be facilitated.

According to the present invention, the at least two supports are pillars extending in a substantially vertical direction. The pillars are adapted for standing on a floor of a medical room. In particular, a pillar may comprise a base with adjustable friction pads for secure standing on the floor. Further, the pillars may have a low-lying center of mass and may be configured for lowering the center of mass of the total support system, including suspended medical devices, in order to further ensure stable standing on the floor. The pillars preferably are configured for transferring the load of the support structure and the medical devices to the floor of the medical room only. No structural connection to the ceiling or a wall of the medical room is necessitated. The medical equipment support system may be configured as a gantry-like structure standing on the floor of the medical room and dimensioned so that a patient can be placed under the support structure, the medical equipment being suspended in a convenient position for medical staff to employ the equipment for performing a medical procedure on the patient. In particular, the medical equipment support system may be configured to be arranged over an operating table on which the patient can be placed, leaving sufficient space between the operating table and the pillars for medical staff to stand or pass in between and sufficient space between the floor and the support structure for medical staff to pass or stand under the support structure, such that unimpeded access to the patient from all sides is permitted.

Due to the supports being configured as vertical pillars for standing on a floor of a room in which the medical equipment support system is placed, the medical equipment support system can be easily retrofitted to any room, without significant changes to the structure of the building being required. In particular, no retrofitting of mechanical support structures and electrical or other connections in the ceiling of the room is necessary. The system can be installed in a medical room without any, or only with a minimum of permanent changes to the existing fabric of the building. In this way it is easy to re-configure a room for a different purpose. For example, with a minimum of changes a room which was once a patient examination room can be changed to an x-ray suite or an anaesthetic bay, etc. The support system can be configured as a non-permanent, adjustable, mobile and/or modular system. The support system in particular allows rapid assembly and removal, adaptability for a variety of applications, ease of transport, storage and cleaning and the possibility of varying functions by virtue of optional accessories. Moreover, due to the support structure being supported in an overhead position, the support system allows suspending medical equipment in its optimal position without impeding access to the patient, providing the advantages of a ceiling-supported mounting system at reduced cost and with increased versatility. The support system may be usable in conjunction with pre-installed overhead lighting and equipment.

According to a preferred embodiment of the invention, the pillars are equipped with wheels and/or gliding elements for moving the pillars and/or the medical equipment support system as a whole on the floor. The wheels may be self-adjusting rollers which may exhibit a blockage device for blocking the wheels when the medical equipment is being used in a medical procedure. Additionally or alternatively, the pillars may be equipped with coupling elements for coupling each pillar to a transport device which in turn may exhibit wheels and/or gliding elements. In this way, transport of the medical equipment support system from one room into another room, as well as moving the system within a room is facilitated.

Preferably, the support structure is removably fixed to the pillars. In particular, the support structure is adapted for being dismantled. The constituent parts of the medical equipment support system, in particular the support structure and the pillars, may each be sized such that transport through the doors of the room where the support system is moving out or in is easily possible. In this way, retrofitting the medical equipment support system to a room where a medical procedure is to be conducted is further simplified.

The support system is adapted for being connected to one or more further support systems. In particular, the medical equipment support system is configured such that the support structure can be coupled to a support structure of a further medical equipment support system in order to increase the number of medical devices that can be carried and/or for increasing the range of positions at which medical equipment can be suspended. In this way, the versatility of the medical equipment support system can be increased.

According to a particularly preferred embodiment of the invention, the at least two pillars are adapted for moving or adjusting the support structure in a vertical direction, i.e. for lowering and/or raising the support structure. The support structure can be fixed in a multiplicity of positions along the vertical direction. In effect, the support structure is height-adjustable. Moving the support structure vertically can be accomplished, for example, by mechanical, electrical, hydraulic, pneumatic and/or other means. In particular, the support structure can be lowered to a level above the floor which permits easy accessibility for maintenance and for mounting or removing medical equipment as well as for cleaning, and can be raised to such a level above the floor that medical equipment can be suspended for easy use by medical personnel, while the support structure is in an overhead position so as not to obstruct access to the patient. In this way, using the medical equipment support system for performing medical procedures, and fulfilling the hygienic requirements in a medical environment, in particular in the environment of an operating room, is facilitated.

Preferably, the pillars and the support structure are configured for accommodating cables and/or fluid lines. The cables may be electric cables for transmission of electric power and/or signals, as well as optical cables for transmission of illumination light or endoscopic images, for example. The fluid lines may comprise, for example, gas supply for insufflation or respiration or lines for irrigation fluid and suction. In particular, the pillars and the support structure may comprise a hollow space wherein the cables and the fluid lines can be installed and which may be accessible by removable hatches. Thus, supply equipment connected to the at least one medical device can be placed away from the support structure, thus avoiding obstruction of available space for medical staff and keeping the supply equipment outside the sterile zone during a surgical procedure.

According to a preferred embodiment, at least one of the pillars comprises a cabinet which is adapted for placing medical equipment in it. In this way the equipment can be made readily available to the medical staff without interfering more than necessary with the medical procedures. In particular, standard medical supply equipment can be placed within the cabinet, being connected to the medical devices suspended from the support structure by cables and fluid lines running through the support structure and the respective pillar.

According to an advantageous embodiment of the invention, the support structure is supported by at least two pillars, each having a strut or foot plate for stable standing on the floor. In particular, the foot plate is enlarged and may comprise sufficient mass to increase stability. Although the strut or foot plate may occupy a greater footprint on the floor, the pillars and foot plates can be positioned where they do not present a great impediment to medical staff. The support structure may comprise, for example, one single elongated beam being supported at each of its ends by one respective pillar. By means of rigid connections between the pillars and the beam, the support system has sufficient stability in its longitudinal direction, and can be prevented from falling in its lateral direction by means of the struts or foot plates. Therefore, the support structure has sufficient stability even if freely standing and needs not necessarily being braced against walls or ceiling. This presents a simple and stable structure for holding medical equipment, being usable in a large number of medical settings and being readily adaptable to the needs in many medical or surgical interventions. Moreover, the foot plates distribute weight over a larger floor area, in many cases avoiding the need for strengthening the floor structure.

According to an advantageous alternative embodiment, the support structure is one beam or a multiplicity of beams arranged parallel to each other, and one end of the beam or of the multiplicity of parallel beams is supported by at least two pillars. In particular, one of the ends of the support structure may be supported by one pillar and its other end supported by two pillars placed at a distance to each other in a lateral or transverse direction with respect to a longitudinal direction of the beam or beams. Preferably, the support structure is supported by two pillars on each of its both ends, the two pillars being at a distance to each other in the lateral direction. The three or four pillars supporting the support structure according to the two aforementioned exemplary configurations define an enlarged footprint of the support system which can ensure that the system itself has adequate stability when freely standing on the floor of a medical room. Stability can be further increased by a cross beam connecting the two pillars supporting one end of the overhead beam or beams at the level of the beam or beams.

Most preferably, the two pillars supporting one end of the beam or beams, being separated in the lateral direction, are rigidly connected to each other, thus forming a rigid pillar unit. The pillar unit itself has a footprint which contributes to stable standing of the support system and to distributing the weight of the system over a larger floor area. The pillar unit may comprise a cabinet which can be configured for accommodating medical supply equipment to be connected to the medical equipment carried by the support system. In particular, the cabinet may be disposed between the two pillars, forming a rigid link between the pillars.

According to the invention, the support structure is at least one beam and/or at least one platform which is configured for supporting at least one holder, the holder being configured for supporting the at least one medical device. The holder may be permanently attached to the support structure or may be attachable to and removable from the support structure. Preferably, the holder is configured for being fixed to the support structure at a multiplicity of positions among which a position can be chosen which fits best to the needs of a particular medical intervention. The holder may permit additional degrees of freedom in holding the medical device and may comprise, for example, an articulated arm and/or a hinge for moving and/or turning the medical device into a convenient position and/or orientation without changing the position at which the holder is held or suspended on the support structure. The holder may comprise, for example, one hinge having a vertical axis to which an articulated arm comprising two hinges having horizontal axes is fixed. The articulated arm may be configured for moving the medical device between an overhead position and a position at about the level of the operating table. The articulated arm may contain sensors and actuators so that it may be automatically positioned. In this way, adaptability to the needs in different kinds of medical interventions can be ensured.

The support structure is formed by one beam or by a multiplicity of beams, which may be arranged substantially parallel to each other, wherein at least one beam comprises a rail, the at least one holder being movable along the rail. The holder may be manually movable, may be motor-driven, or may be automatically movable under computer control. Thus, for example, suspended medical equipment may be moved or may move automatically back and forth during a medical procedure to deliver or remove equipment as required. The holder itself may also be configured to be automatically adjustable, for example the holder may comprise a robotically controlled arm which can pick-up and return equipment stored in a carousel or other predetermined spatial arrangement. The holder may be configured for being fixed at a position on the rail that can be chosen to fit the needs of a particular medical intervention. Thus, for example, the support structure may consist of two parallel beams, each beam comprising a rail to which one or several holders are attached being movable along the respective rail. Preferably, each holder is configured for being fixed to the rail at one or a multiplicity of positions along the rail. Due to the holders being movable along the rail, versatility and ease of use of the support system can be further increased. In particular, due to the weight of the medical equipment being carried by the support structure, transport of medical equipment is facilitated. Moreover, unused spaces of a medical room such as near the ceiling can be employed for storage of medical equipment, and suspended equipment can easily be stored in a storage area or removed from the storage area when needed. On the other hand, the floor can be kept clear, and when a part or all of the equipment is not in use it does not take up valuable floor space.

While a support structure consisting of one overhead beam or two or more parallel beams offers the advantages of versatility and simplicity, other configurations of the support structure may be employed if suitable in a particular setting or for a particular procedure, such as, for example, circular, triangular, rectangular, or hexagonal or irregular platforms. Correspondingly, curved rails for holding and moving medical equipment holders may be provided.

In cases in which the support structure comprises at least one overhead rail for moving equipment, a device may be mounted on the rail which is able to clean the support structure, in particular an overhead beam, while moving along it. This device may incorporate brushes and cleaning fluid and may be either wholly automatic, self-propelling or manually controlled.

The at least one holder is movable into at least one preference position which is defined within a predetermined medical scheme. In particular, the medical scheme may be a scheme of positions and orientations of medical devices required in a particular medical procedure, for example in a particular surgical procedure. The positions and orientations of the medical devices may be such positions and orientations for operating the medical devices and/or for loading, unloading or cleaning the medical devices. In order to reach the preference position, the holder may be movable along the rail and/or the holder itself may be adjustable with respect to its own degrees of freedom. By moving the holder into the preference position, the medical device carried by the holder is placed in a preference position and orientation as determined by the medical scheme. There may be more than one preference position and orientation for each medical device. In this way moving the medical devices required in a particular medical procedure into positions and orientations required by the medical procedure is facilitated.

According to a preferred embodiment, the at least one holder is manually movable, the at least one preference position being defined by a stop. In particular, the stop may be haptically sensible when the holder is manually moved along the rail. The haptic sensing of the stop can be achieved by an increased resistance or a detent being provided on the rail at the preference position. The position of the stop may be automatically adjustable, in particular the stops may be powered, so that they can be moved automatically into position under computer control. The stop or stops may be designed to allow some holders to pass unhindered and stop only specific, predetermined holders which may be configured for holding a particular kind of medical equipment. In a similar way, a stop may be provided within the holder if the holder is adjustable in itself. By means of the manual movement, the position and orientation of the medical devices can be controlled by the medical staff in an optimal manner, while reaching of a predetermined preference position is facilitated.

According to the invention, the at least one holder is automatically movable into the at least one preference position. This may be achieved by means of a drive system and a sensor system. The drive system is configured for moving the holder along the rail and, if the holder provides one or more degrees of freedom, for adjusting the holder itself. The sensor system is configured for detecting when the preference position has been reached. A control system may be provided for controlling the drive system and the sensor system such that when the preference position has been reached the drive system is automatically stopped. Preferably, the control system is also configured for controlling the medical devices used in the medical procedure. Due to the at least one holder being automatically movable into the at least one preference position, an optimal arrangement of medical devices can be most easily achieved before and during a medical procedure.

In particular, the at least one holder may be automatically movable into a multiplicity of predetermined preference positions. For example, a first set of preference positions, in which the holder is positioned prior to a medical procedure, may be in an adjoining equipment room where the holders can be loaded with the equipment appropriate to the anticipated medical procedure. A second set of preference positions would be in the operating room, being defined within the medical procedure as described above. A third set of positions, employed at the end of the medical procedure, would carry the equipment to a waste and cleaning area for appropriate disposal. The holders may be automatically movable into the first, second, and third sets of preference positions, according to the progress of the medical procedure, by means of drives, sensors, and a control system as described above. In this way, handling of medical devices can be facilitated before, during, and after a medical procedure.

According to a preferred embodiment of the present invention, the medical equipment support system is configured for supporting a first multiplicity of medical devices mounted to a first multiplicity of holders and for supporting a second multiplicity of medical devices mounted to a second multiplicity of holders. The system is configured for automatically removing the first multiplicity of holders from the vicinity of the operating table and for automatically moving the second multiplicity of holders into the vicinity of the operating table. In this way, the first multiplicity of medical devices can be automatically replaced with the second multiplicity of medical devices. Preferably, the second multiplicity of holders may be automatically moved into respective preference positions which may correspond to a predetermined medical scheme. In particular, the first multiplicity of medical devices may correspond to a first medical scheme, and the first multiplicity of holders may be arranged at respective first preference positions corresponding to a first predetermined medical scheme, and the second multiplicity of medical devices corresponds to a second medical scheme, the second multiplicity of holders being automatically arranged in respective second preference positions corresponding to a second predetermined medical scheme. Upon a signal entered by medical staff, the first multiplicity of devices can be automatically removed from the vicinity of the operating table and moved into a storage area or room, and the second multiplicity of devices are automatically transported from a storage area into the vicinity of the operating table, being moved into positions corresponding to the respective second preference positions of the holders. The first and second holders themselves may provide additional degrees of freedom permitting further adjustment of position and/or orientation of each medical device, which may be automatically adjusted in a similar way. Positions and/or orientations of holders and medical devices may be pre-defined according to first and second medical schemes. For transport into and from the storage area, the one or multiple rails to which the holders are attached may exhibit an extension or respective extensions or connections to other support systems.

In particular, the first multiplicity of devices may be surgical devices required in an endoscopic intervention or "keyhole surgery" (Minimal Access Surgery, MAS), while the second multiplicity of devices may comprise surgical devices required for corresponding open surgery. When, in performing a MAS procedure, a situation occurs which requires rapid conversion from MAS to open surgery, such as sudden severe bleeding, the medical devices employed for MAS can be quickly and safely replaced by those devices required in a corresponding open surgery procedure. Thus, the ability of surgical staff to cope with unexpected events or emergency situations can be increased.

More generally speaking, equipment can be stored some distance from the point of use and moved automatically to and from storage along the overhead rail or an extension of the rail. A control unit of the system may be pre-programmed to automatically deliver equipment to an operating theatre in "just-in-time" fashion, according to a specific treatment schedule. Similarly, medical equipment may be automatically removed from the operating theatre, after having been used. When the operating theatre is prepared for another surgical procedure, clutter of the workspace and excessive delays associated with awaiting the delivery of equipment from distant storage can be avoided in this way.

Preferably, the system design is modular so that it can be tailored to the specific requirements and dimensions of a particular operating theatre. Each module is small enough to be transported through the doors of the theatre, after which the modules may be assembled in situ. The modules include the pillars, optionally with a built-in raising and lowering mechanism, and one or more overhead mounting beams, which may be cut to the desired length. Moreover, the modules may include one or more equipment cabinets, stabilising supports, holders with adjustable positioning arms capable of mounting various equipment, one or more cross-beams for increasing lateral rigidity, and, possibly, one or more extensions for storage of equipment in a storage area or storage room. In particular, the modules are designed to be releasably connectable to each other and to be transportable into an operating theatre through standard access doors. In this way, optimal versatility of the support system can be achieved.

The features of the invention as mentioned above and as described below apply not only in the combinations mentioned but also in other combinations or alone, without leaving the scope of the present invention.

Further aspects of the present invention will be apparent from the figures and from the description of particular embodiments that follows.
Figs. 1a and 1b show a first embodiment of the invention in a side view and in a perspective view in an operating theatre, respectively, and Fig. 1c shows a variation of the first embodiment;
Fig. 2 illustrates a second embodiment of the invention in a perspective view in an operating theatre;
Figs. 3a and 3b show a third embodiment of the invention in two positions in a perspective view in an operating theatre;
Figs. 4a - 4e depict a fourth embodiment of the invention and further details;
Fig. 5 shows an alternative embodiment of a beam and mounting block;
Fig. 6 illustrates an elevation mechanism in a schematic side view;
Figs. 7a and 7b show a fifth embodiment of the invention;
Fig. 8 illustrates a variation of the fifth embodiment;
Fig. 9a - 9f show three examples of transport means for the support system.

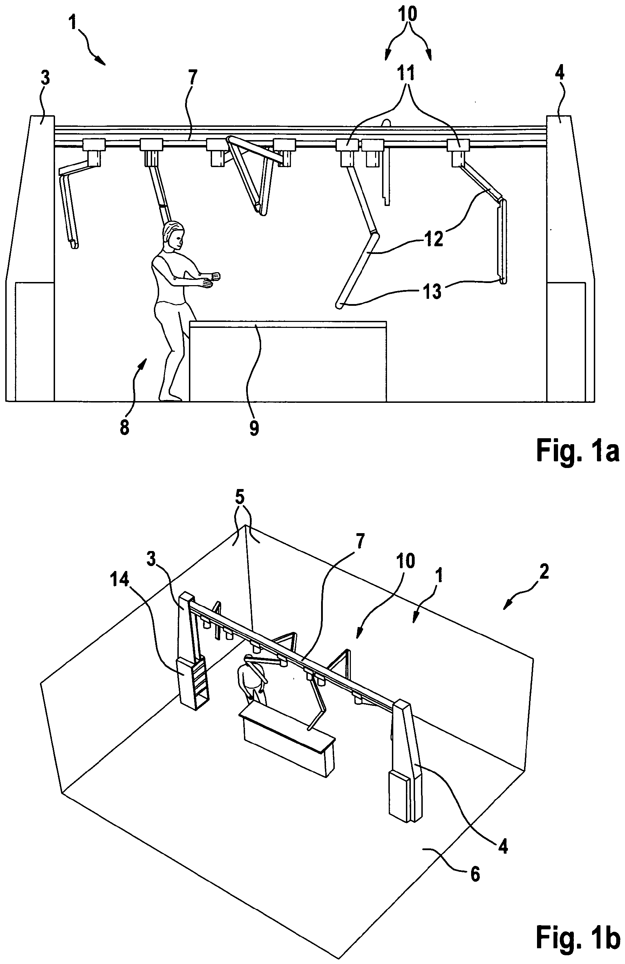

As shown in Figs. 1a and 1b, a medical equipment support system 1 according to a first embodiment of the invention is set up in a medical room, which may be an operating theatre 2. The support system 1 comprises two vertical pillars 3, 4 standing at a distance to the walls 5 on the floor 6 of the operating theatre 2. The pillars 3, 4 are connected close to their top ends to a horizontal beam 7, thus supporting the beam 7 in an overhead position with respect to medical staff 8. The beam is at such a height that medical staff 8 can comfortably pass or stand under it, for example at about 2.5 m above the floor 6. The pillars 3, 4 and the beam 7 form a gantry passing over an operating table 9. The pillars are spaced sufficiently that medical staff 8 has free access from all sides to the operating table 9, for example the pillars are spaced in a horizontal direction by about 4-5 m. Attached to the beam 7 are a number of holders 10 each comprising a mounting block 11 and an articulating arm 12 onto which suitable medical equipment may be mounted. In particular, one or several medical devices may be mounted close to the lower end 13 of each articulating arm 12. For example, a monitor, lighting, a surgical instrument tray and supplies of medical gasses, suction, irrigation and power for electrosurgery could be mounted to the articulating arms 12 and held in a position close to the operating table 9 if currently in use in a surgical procedure, or in a storage position if currently not in use. Such medical devices are not shown in the figures.

Pillar 3 contains a cabinet 14 sized so that standard medical equipment may be placed in it. Possibly, both pillars 3, 4 each contain such a cabinet. Equipment in the cabinet 14 may be linked to the articulating arms 12 via the overhead beam 7 so that, for example, an insufflation pump placed in cabinet 14 may have a tube running through pillar 3 and overhead beam 7, block 11 and arm 12 to the patient. An easily accessible space for routing cables and tubes through the beam 7 and the arms 12 may be provided for this purpose (see below).

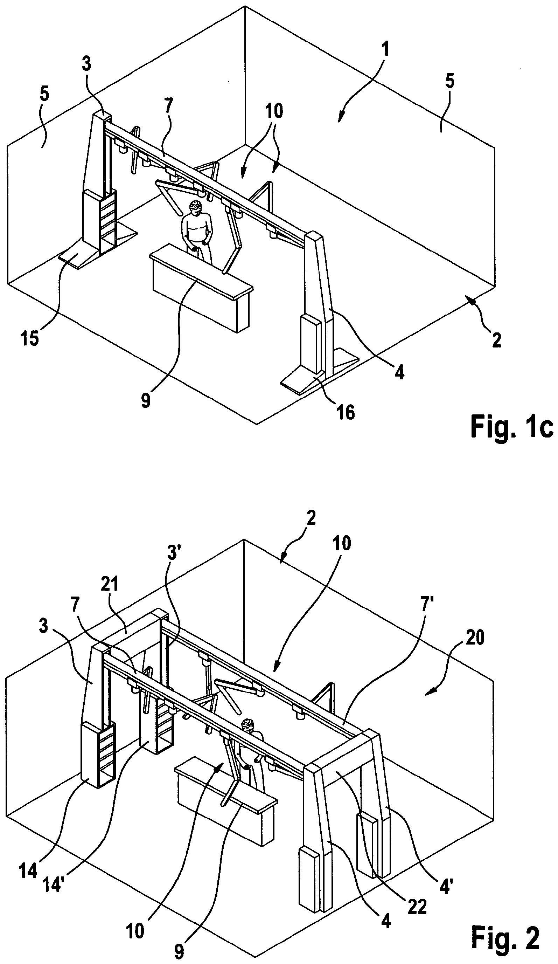

The pillars 3, 4 each have a footprint which is large enough to ensure sufficient stability to the gantry. According to a variation of the first embodiment and as illustrated in Fig. 1c, the pillars 3, 4 each may comprise a foot plate 15, 16 providing an enlarged footprint for increased stability. Additionally or alternatively, at least one of the pillars 3, 4 may be non-permanently connected to a wall 5 of the operating theatre 2 to increase stability (not shown).

According to a second embodiment of the invention and as depicted in Fig. 2, a medical equipment support system 20 comprises two parallel overhead beams 7, 7'. A first beam 7 is supported at its first and second ends by two pillars 3, 4, and the second beam 7' is supported at its first and second ends by two pillars 3', 4'. The adjacent pillars 3, 4 supporting the two beams 7, 7' at their first ends are rigidly linked to each other close to the connection to the beams 7, 7' by a lateral cross beam 21, and the pillars 3', 4' supporting the two beams 7, 7' at their second ends are rigidly linked to each other by a lateral cross beam 22. Thus, due to a total of four pillars 3, 3', 4, 4' placed in a rectangular arrangement and rigidly connected in pairs a structure is created that provides increased stability. With respect to other features, the second embodiment is configured like the first embodiment described above. In particular, each of the beams 7, 7' may be equipped with one or more holders 10 to which medical equipment can be mounted, and one or more of the pillars 3, 3', 4, 4' may comprise a cabinet 14, 14'. During use, the support system 20 will preferably be placed so that the operating table 9 is arranged under the rectangle formed by the beams 7, 7' and the cross beams 21, 22, so that surgical equipment can be suspended from all sides towards a patient placed on the operating table 9.

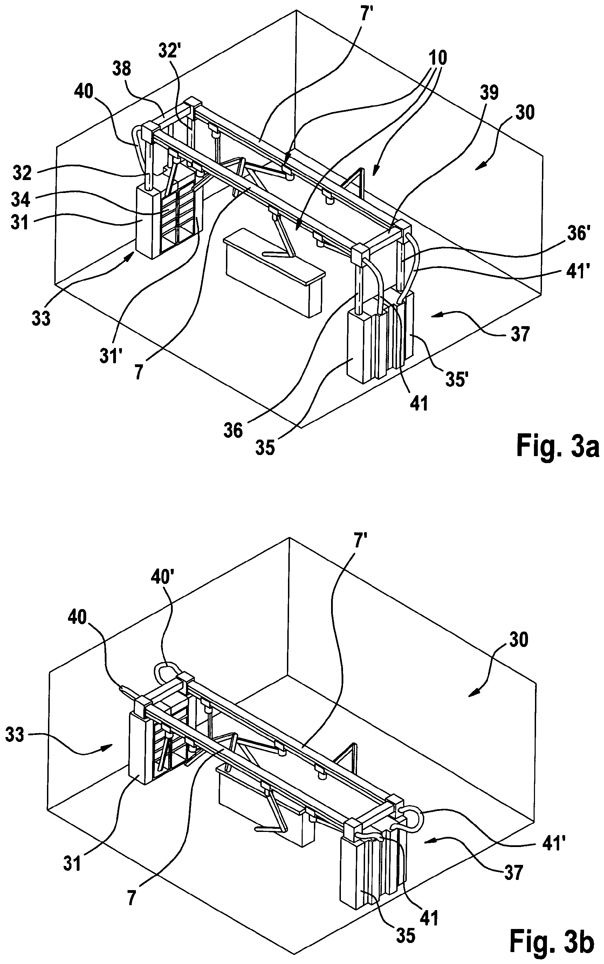

A third embodiment of the invention is shown in Figs. 3a and 3b. According to this embodiment, a medical equipment support system 30 generally corresponds to the second embodiment, but each of the four pillars 31, 31', 35, 35' supporting the beams 7, 7' comprises a height-adjustable shaft 32, 32', 36, 36' carrying one end of a respective beam 7, 7'. Moreover, the two adjacent pillars 31, 31' supporting the first ends of the beams 7, 7' are connected to each other to form a pillar unit including a pillar base 33 with a cabinet 34. The two pillars 35, 35' supporting the two parallel beams 7, 7' on their second ends form a unit comprising a pillar base 37 which also may include a cabinet. At their upper ends, the shafts 32, 32' carrying the first ends of the beams 7, 7' are connected to each other by a cross beam 38, and the shafts 36, 36' are connected to each other by a cross beam 39.

The shafts 32, 32', 36, 36', and consequently the beams 7, 7', can be raised and lowered by means of an elevation mechanism included in each pillar 31, 31', 35, 35'. While Fig. 3a shows the support system 30 in an overhead position in which the equipment mounted to the support system 30 via equipment holders 10 is ready for use in a surgical procedure, the support system 30 can be lowered to a low position as shown in Fig. 3b. In this way easy cleaning, installation and maintenance of equipment which is attached to the holders 10 is allowed. In order to permit routing cables and tubes from the pillar bases 33, 37 to the beams 7, 7', flexible guide hoses 40, 40', 41, 41' may be employed with sufficient lengths to account for the raising and lowering. Methods and components for moving the shafts 32, 32', 36, 36' to raise and lower the beams 7, 7' are described below.

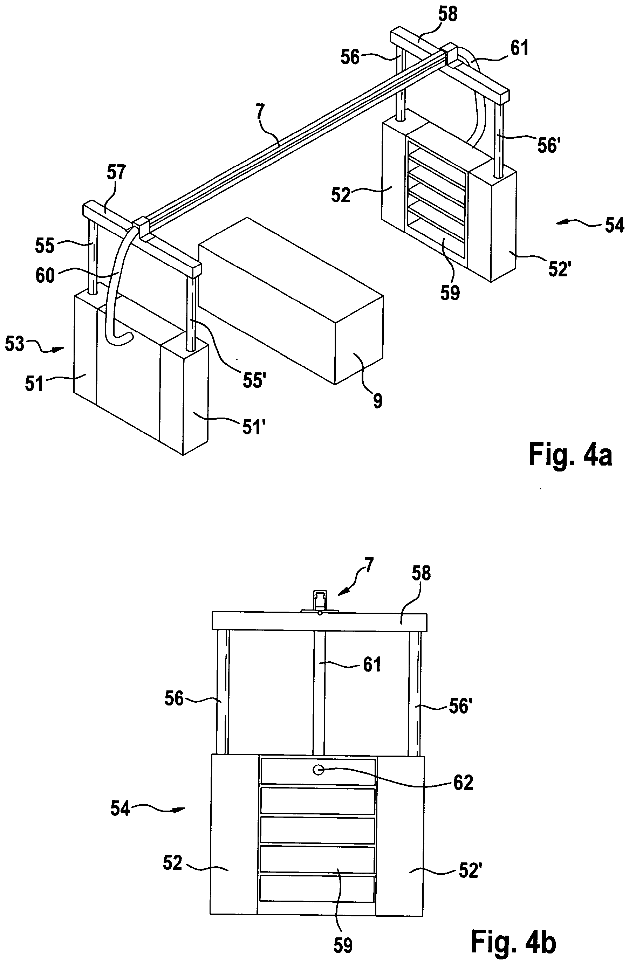

According to a fourth embodiment of the invention and as shown in Fig. 4a, a medical equipment support system 50 comprises two pillar units each comprising two pillars 51, 51', 52, 52' connected to each other to form a respective pillar base 53, 54. Each of the pillars 51, 51', 52, 52' comprises a height-adjustable shaft 55, 55', 56, 56' as described above with respect to the third embodiment. At their top ends, each two adjacent shafts 55, 55', 56, 56' are joined by a respective cross beam 57, 58. The cross beams 57, 58 support a single horizontal beam 7 connected to the respective cross beam 57, 58 approximately at half-length of the cross beam 57, 58. At least one of the pillar units is equipped with a cabinet 59. Flexible guide hoses 60, 61 connect both ends of the horizontal beam 7 to the pillar bases 53, 54, accounting for an elevation of the horizontal beam 7 due to vertical movement of the shafts 55, 55', 56, 56'. The cables and tubes running from the pillar bases 53, 54 to the mounting beam 7 must allow sufficient slack to avoid breakage when the beam 7 is raised and lowered. This is achieved by routing the cables and tubes through the guide hoses 60, 61 which protect the cables and tubes while allowing them to flex and additionally makes the bundle of cables and tubes easy to clean. Medical equipment is mounted directly to the beam 7 or to holders which in turn are attached to the beam 7, as described above. The holders are not shown in Fig. 4a. As indicated symbolically in Fig. 4a, in use the operating table 9 may be arranged under the beam 7 in a convenient orientation with sufficient free space around.

Fig. 4b depicts details of a pillar unit comprising pillar base 54 and vertically adjustable shafts 56, 56' as described above. The pillar base 54 contains a cabinet 59 that allows medical equipment to be stored on adjustable shelving. The equipment cabinet 59 provides a structurally rigid link between the pillars 52, 52' and spreads the loading from the structure over a larger floor area. To reliably transfer structural loads, a strong connection is desirable between the pillars 52, 52'. Bolt holes may be provided for this purpose so that the pillars 52, 52' and the equipment cabinet 59 may be bolted together.

Moreover, it is desirable to link equipment in the cabinet 59 to equipment carried by the overhead mounting beam 7. The equipment in the cabinet 59 in turn may be connected to external supplies of electricity, fluid, and vacuum. Thus, for example, a monitor on the overhead beam 7 or on a holder attached to the overhead beam 7 may be linked to an endoscopic video image source located in the cabinet 59, or an insufflation pump in the cabinet 59 may have its tubing routed through the overhead beam 7 to the patient. In order to achieve this, a port 62 is provided at the rear of the equipment mounting cabinet 59, and a second port is located at the end of the equipment mounting beam 7. Tubes and cables may easily be fed through these ports when the beam 7 is in its low position. When in use, the bundle of cables and tubes passing from the equipment cabinet 59 to the overhead beam 7 is contained in the flexible hose 61 to protect the bundle and make it easier to clean. Alternatively or additionally, the cables and tubes may be covered by a length of flexible conduit which, during installation of the cables and/or tubes, may be compressed back to a short length and secured over a tubular retaining feature so that cables and/or tubes may be easily passed through it (not shown in the figures). The conduit may be constructed from helical wire-reinforced tubing, which is able to collapse to a short length.

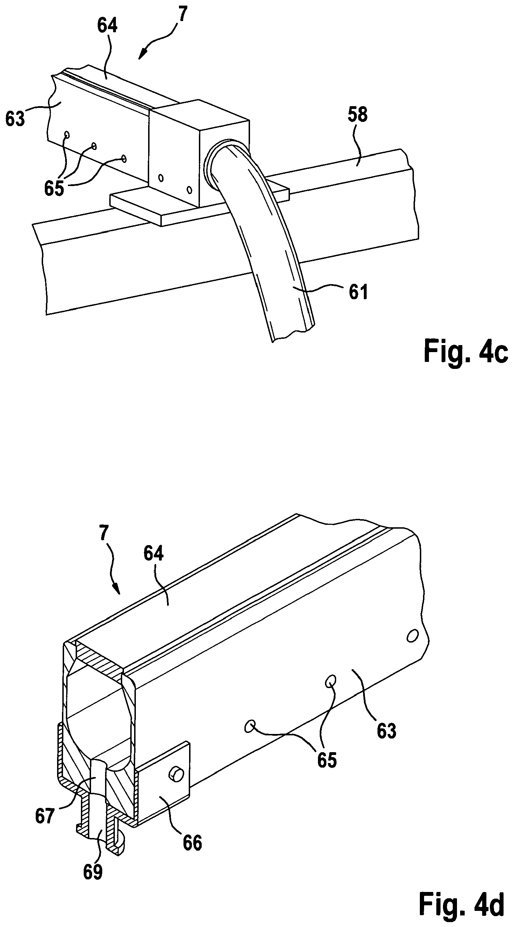

In Figure 4c details of the hose or conduit connection to the overhead mounting beam 7 are shown. The cross beam 58 connects the shafts 56, 56' to the equipment mounting beam 7 (see Figs. 4a, 4b). The cross beam 58 is a hollow aluminium extrusion, with its ends closed by caps to ensure that it is easy to clean. The equipment mounting beam 7 comprises a channel profile 63, preferably a U shaped aluminium extrusion. The mounting beam 7 is cut to length to match the size of the operating theatre where the support system 50 is to be placed. The top of the U section is covered with a plastic cover 64. The cover 64 seals the beam 7 from contamination, making it easy to clean, but may be temporarily removed in order to allow routing the cables or tubes through the beam 7. The beam 7 is drilled with a series of horizontal holes 65 at regular intervals. The horizontal holes 65 act as attachment points for medical equipment, usually via a positionable equipment mounting block, to which an articulating arm may be attached on which in turn at least one medical device can be mounted. Bolts can be passed though the horizontal holes 65 for a secure mount of the mounting block and due to their regular spacing there are numerous possible positions for attachment.

In Figs. 4d and 4e, the horizontal equipment mounting beam 7 is shown in two different perspective views, wherein Fig. 4d shows a cross section, and Fig. 4e is a view from below. The cross section of the profile 63 allows for easy cable routing through its interior. Also shown is an equipment mounting block 66 which is retained by two transverse bolts. It includes an aligned hole for cable and/or tube routing into an arm attached to the mounting block 66 (not shown). As can be seen in Figs. 4d and 4e, vertical holes 67 allow the routing of cables or tubing out of the beam 7 through the mounting block 66 into an equipment mounting arm.

For this purpose the mounting block has a hole 69 which is aligned with one of the vertical holes 67. The majority of the horizontal holes 65 and vertical holes 67 will not be in use at a given time, so plastic caps for the holes may be provided to ensure that the inside of the beam 7 remains sealed and to aid cleaning.

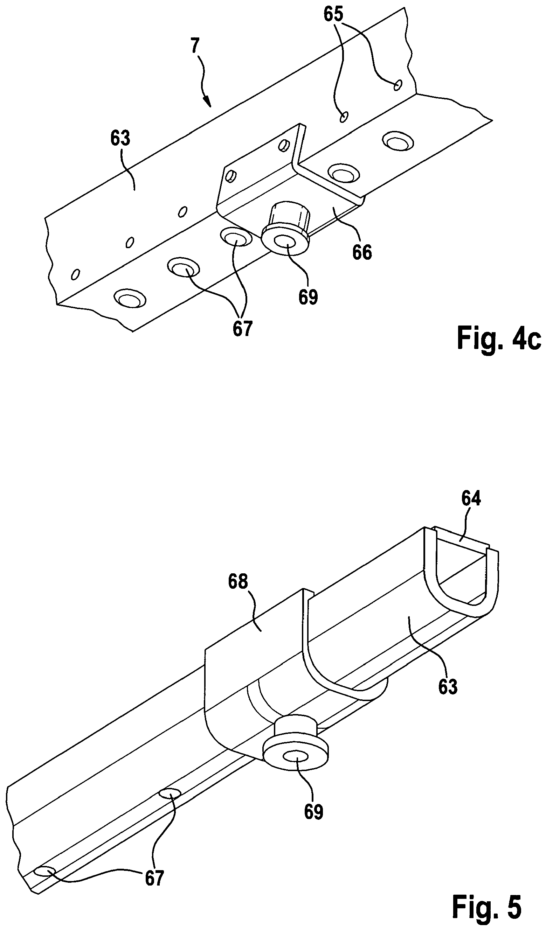

Alternatively, as shown in Fig. 5, a mounting block 68 may be provided which is movably held on the mounting beam 7. The beam 7 is formed by a U shaped profile 63, which is covered on its top by a cover 64, which may be removed in order to route cables along the channel formed inside the profile 63. The cross-section of the mounting block 68 fits that of the profile 63, encompassing it for guidance along the beam 7. A series of vertical holes 67 are drilled through the bottom of the profile 63. When not in use, the holes are covered by caps, but they may also be used to route cables or tubes out of the beam. An equipment mounting arm can be attached to the beam 7 by the movable mounting block 68. The mounting block 68 includes a hole 69 which may be aligned with the holes 67 in the bottom of the profile 63 to allow cable or tube routing to the equipment. Moreover, fixation means may be provided for fixing the movable mounting block 68 in any position on the beam 7 (not shown).

The design details depicted in Figs. 4b through 4e and in Fig. 5 have been described with respect to the embodiment shown in Fig. 4a, but may pertain as well to the other embodiments described, in particular to embodiments comprising two parallel arms 7, 7' (see Figs. 3a, 3b). Preferably, all electrical wiring and tubes are routed inside the components of the system in order to improve cleanability. Optionally, the articulating arms for mounting medical equipment also incorporate a channel through which cables and tubes may be routed.

Raising and lowering the beams 7, 7' (see Figs. 3a, 3b, 4a, 4b) may be achieved using various linear actuators, using power provided by the use of hydraulic rams, motors or manually driven cranks, including, for example, a system of pulleys or rack-and-pinion systems. Such mechanisms are well-known in themselves and are therefore only briefly described.

In one embodiment of a raising and lowering mechanism, a hydraulically actuated cylinder is located in each pillar 31, 31', 35, 35', 51, 51', 52, 52' so that the height of the shaft 32, 32', 36, 36', 55, 55', 56, 56' of the respective pillar may be adjusted. An electrically driven hydraulic pump located in one of the pillar bases 33, 37, 53, 54 provides power to the system. Preferably, the cylinders are controlled so that they are constrained to raise and lower at the same rate, so that the equipment mounting beam 7, 7' always remains horizontal and, in embodiments with two parallel beams 7, 7', both beams 7, 7' always are held at the same elevation.

In another embodiment, the height of the pillars 31, 31', 35, 35', 51, 51', 52, 52' is adjustable by electrically driven lead screws or ball-screws. Each lead or ball screw is connected via a nut to the shaft 32, 32', 36, 36', 55, 55', 56, 56' of the respective pillar which is free to slide vertically within the pillar, but is otherwise constrained. A screw shaft is driven by an electric motor in the base of the respective pillar 31, 31', 35, 35', 51, 51', 52, 52' via a gearbox so that the electric motor can rotate the screw shaft, causing the nut and the shaft 32, 32', 36, 36', 55, 55', 56, 56' connected to the nut to move upwards or downwards. The pillars 31, 31', 35, 35', 51, 51', 52, 52' may be sensorised so that their height can be monitored during operation and controlled with a closed loop system, or stepper motors may be used. This ensures that all pillars 31, 31', 35, 35', 51, 51', 52, 52' are raised together, keeping the equipment mounting beam 7, 7' horizontal regardless of loading. During assembly of the modules the pillars 31, 31', 35, 35', 51, 51', 52, 52' may be synchronised. The pitch of the screw can be chosen to be passively self-locking, so that a hazardous failure is unlikely.

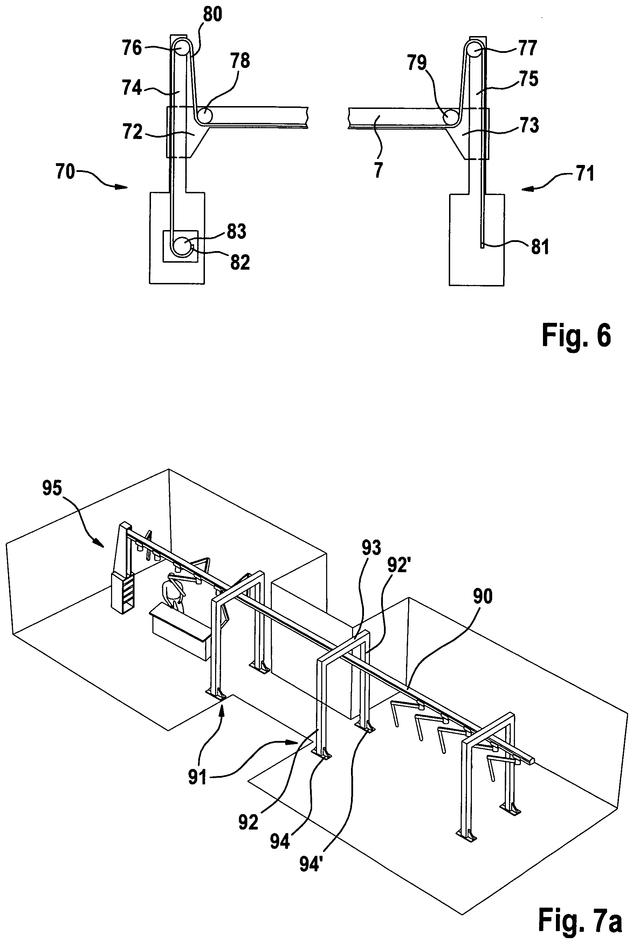

In still another embodiment, a chain or cable is used to raise or lower the pillars. In this configuration which is schematically shown in Fig. 6, the pillars 70, 71 are of fixed height, while the mounting beam 7 is free to slide up and down the pillars 70, 71. The beam 7 is constrained in its angle to the pillars 70, 71 by guide sleeves 72, 73 fixed to the ends of the beam 7, each guide sleeve 72, 73 being freely movable along a stationary guide shaft 74, 75 of the respective pillar 70, 71. In effect, the beam 7 remains perpendicular to the pillars 70, 71. A respective upper pulley 76, 77 is located at the top of each pillar 70, 71 and a respective lower pulley 78, 79 at each end of the mounting beam 7. A first end 81 of a chain or cable 80 is fixed to one pillar 71 while a second end 82 is fixed to a drum 83 connected to a motor via a gearbox (not shown). The motor can be used to rotate the drum, and thus raise and lower the beam 7. Alternatively, a crank handle may be used instead of the motor so that the mounting beam 7 may be raised and lowered manually. Preferably, a worm gear arrangement is used to ensure that the system is self-locking, but alternatively this could be achieved with a ratchet mechanism. The advantages of this embodiment are that a single motor may be used to raise and lower the beam 7, while the beam 7 is passively maintained in a horizontal orientation.

For all embodiments, it may be desirable to include a failsafe feature such as a bolt to lock the structure in place when in use, so that it may not under any circumstances collapse.

The medical equipment may be attached to the equipment mounting beam via sliding attachment which may travel along the overhead horizontal beam. Such sliding attachment may be configured, for example, as a movable mounting block 68 as depicted in Fig. 5. Optionally wheels or bearings may be used to reduce friction. When in use, the sliding attachment is locked in position, but when not in use it allows the repositioning and transport of attached medical equipment along the beam 7.

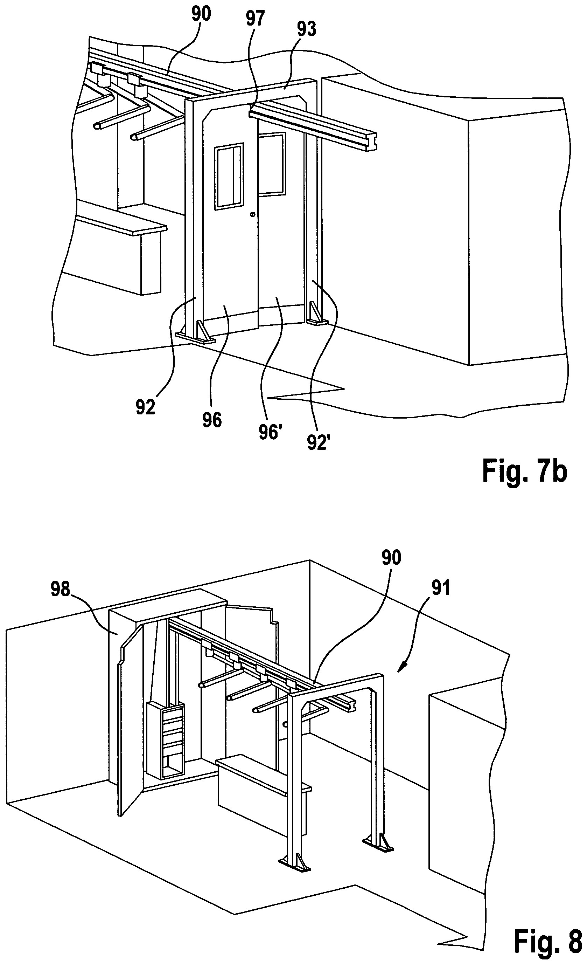

To facilitate the transport of equipment from one area to another, an extension of the overhead beam may be used which passes through an existing doorway from one room to another, for example into an equipment storage room, as shown in Figs. 7a and 7b. As can be seen in Fig. 7a, the extended beam forms an overhead track or rail 90 linking a storage area (right) and an operating theatre (left). The overhead rail 90 may even extend along corridors or passageways, and provision may be made for means of negotiating the lintel above doorways. Possibly, a multiplicity of medical equipment support systems may be interlinked with, if required, computer control. The overhead rail 90 is suspended from a series of intervening portal-shaped supports 91 each formed by two pillars 92, 92' linked by an overhead cross beam 93, the pillars 92, 92' standing on the floor, each having a foot plate 94, 94' for secure standing. This provides for ready transportation of suspended items between use and storage. The left-hand part of the system may be configured as described above, in particular, the rail 90 may be held by a pillar 95 which is configured as described above.

Doors which may be provided between the storage area and the operating theatre are omitted in Fig. 7a. Doors present important physical barriers in infection control in a medical treatment setting. Advantageously, such doors can be incorporated into the gantry supports, as illustrated in Figure 7b. In this case, the opening defined by the pillars 92, 92' and the cross beam 93 is filled by a double wing door, the door wings 96, 96' being cut out to form a tightly fitting passage 97 for the rail 90. In Figs. 7a and 7b some of the walls and the ceiling of the rooms are not shown.

In situations where a separate storage room is unavailable, an alternative means of storing the medical equipment would be to configure the pillar 95 on the far left of Fig. 7a as a storage cabinet 98 which also serves as a support for the rail 90. A simple example of this embodiment is illustrated in Fig. 8.

In any one of the embodiments described, the design may be modular so that it can be tailored to the specific requirements and dimensions of a particular operating theatre. All modules of the system are sufficiently small that they may be transported into an operating theatre through the standard access doors, and the components can be assembled relatively quickly in situ. The modules may be the pillars 3, 3', 4, 4', 31, 31', 35, 35', 51, 51', 52, 52', 70, 71, 92, 92', 95, the mounting beam or beams 7, 7', the cabinets 14, 14', 34, 59, 98, and, possibly, further stabilising supports, articulating arms 12 for mounting medical equipment, a beam extension for storage of equipment, and the cross beams 21, 22, 38, 39, 57, 58. The overhead beam 7, 7' or the rail 90 may be available in a range of lengths so that it could be easily tailored to the size of the room; if necessary the beam 7, 7' or the rail 90 can be assembled from multiple parts in situ. Modules are designed to fit together and then be secured by bolts or other releasable mechanical fasteners so that they may be dismantled or reconfigured at a later date. The support system remains in place as long as required but may be readily removed or adjusted to permit the use of a given room or space to be altered without permanent structural or architectural alterations to the building. All connections preferably are non-permanent so that the assembled structure can be disassembled for transport or reconfiguration at a later date.

For example, to produce a system as shown in Figs. 3a and 3b, two equipment mounting beams 7, 7' are selected to suit the dimensions of the operating theatre. These are combined with equipment cabinets 34, four pillars 31, 31', 35, 35' and two cross-beams 38, 39 along with a multiplicity of equipment holders 10. The cabinets 34 and pillars 31, 31', 35, 35' are bolted together so as to form pillar bases 33, 37 providing stable structures and spreading the loading across a larger floor area. At its simplest, the modular medical equipment support system could be used with a single equipment mounting beam 7, two fixed pillars 3, 4 and, possibly, foot plates 15, 16 to spread the load and stabilise the pillars 3, 4, as illustrated in Figs. 1a through 1c.

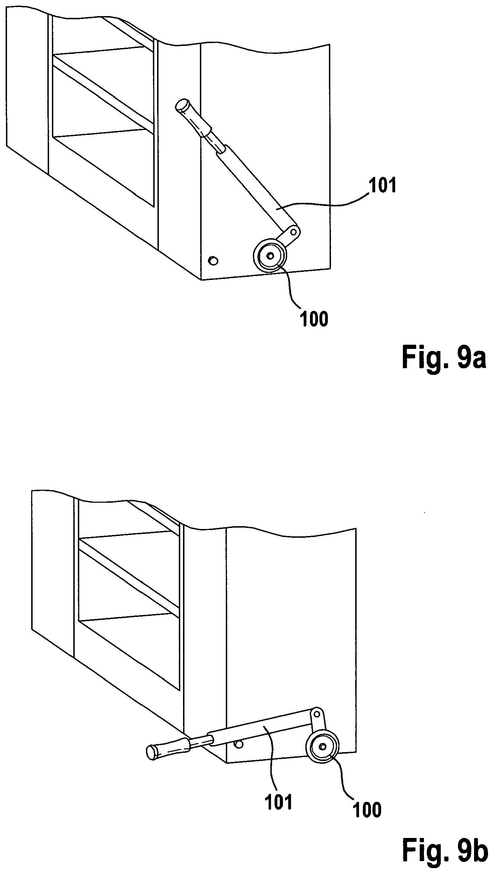

To aid the transportation of heavy or bulky modules or subassemblies or even the support system as a whole, the pillars 3, 3', 4, 4', 31, 31', 35, 35', 51, 51', 52, 52', 70, 71, 92, 92', 95 and/or pillar bases 33, 37, 53, 54 described above may optionally be equipped in one of the ways illustrated in an exemplary manner in Figs. 9a through 9f:
As shown in Figs. 9a and 9b, the pillars or pillar bases may be equipped with wheels 100 which may be retracted when the medical equipment support system is in its position for use (Fig. 9a) and extended by means of a lever 101 when required for transportation (Fig. 9b). The lever 101 may act upon another wheel located on the opposing side of the pillar base which is not visible in Figs. 9a and 9b. The lever 101 may be designed such that its extended position, i.e. the active position of the wheels 100, is self-locking.

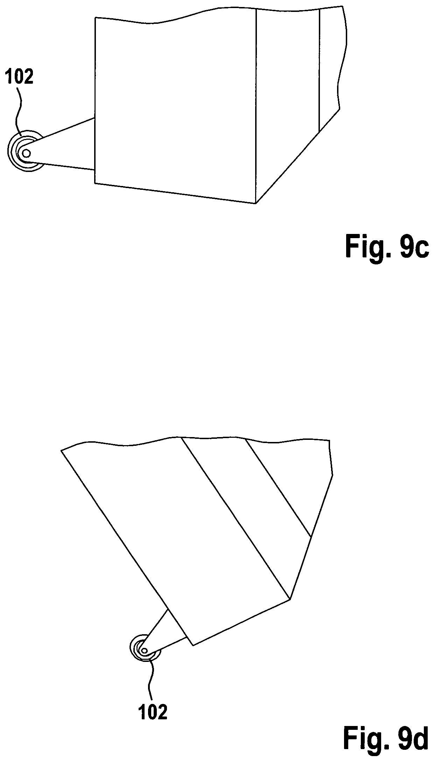

Alternatively, each pillar or pillar base may have one or several wheels 102 placed such that they bear the weight of the pillar or pillar base when it is tilted (see Fig. 9d). When it is not tilted, the wheels 102 are raised slightly above the floor (Fig. 9c).

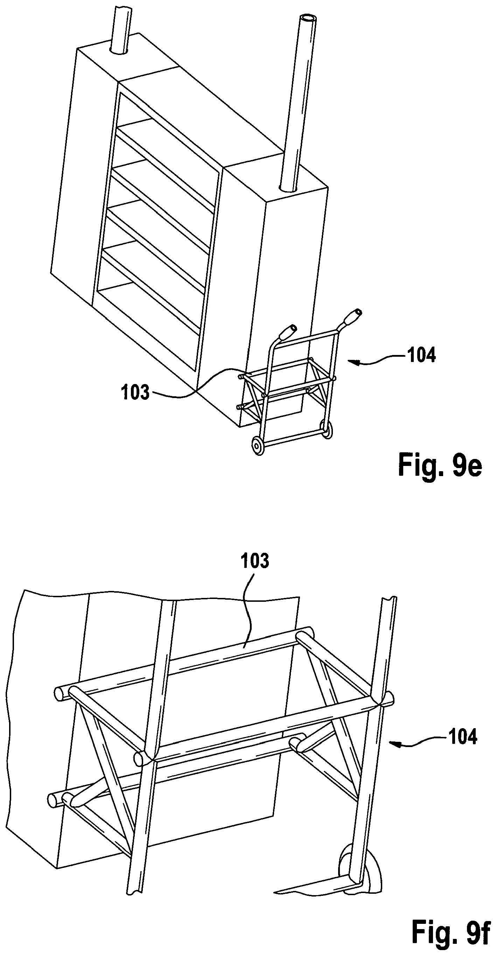

Alternatively or additionally, the pillar or pillar base may exhibit features which engage in a dedicated transport trolley to facilitate uplifting (see Figs. 9e and 9f). Such features may include bars 103 or hooks designed to secure the component to a trolley 104 when it is lifted.

In any one of the embodiments described, medical equipment carried by the medical equipment support system 1 on the beam 7, 7' or rail 90, either directly or via a holder 10, may be, for example, lighting, power sockets or socket posts which provide multiple cable plug-ins, connections to vacuum, insufflation and medical gasses, irrigation, fluid drainage, electrosurgery devices and anaesthetics, instrument holders, floating table, instrument tray, visual display unit (monitor), raised storage shelves, patient hoist, armrest and/or seat, isolation transformer, operating microscope, patient monitoring equipment such as pulse oximeter, electrocardiography, etc. and/or imaging equipment such as for fluoroscopy. Even the operating table may be suspended from the support structure. Moreover, the medical equipment support system 1 may be used in conjunction with overhead lighting and equipment and overhead attachments for drapes, covers or curtains installed on the ceiling of the operating theatre 2.

While the embodiments of the invention have been described in relation to use in an operating theatre, the medical equipment support system may be employed as well in other care-giving settings such as treatment rooms, hospital wards, imaging facilities, storage areas, etc.

For clarity not all reference numerals are displayed in all figures. If a reference numeral is not explicitly mentioned in the description of a figure, it has the same meaning as in the other figures.

### Reference numerals

- 1: Support system
- 2: Operating theatre
- 3, 3': Pillar
- 4, 4': Pillar
- 5,5': Wall
- 6: Floor
- 7, 7': Beam
- 8: Medical staff
- 9: Operating table
- 10: Holder
- 11: Mounting block
- 12: Articulating arm
- 13: Lower end
- 14, 14': Cabinet
- 15: Foot plate
- 16: Foot plate
- 20: Support system
- 21: Cross beam
- 22: Cross beam
- 30: Support system
- 31, 31': Pillar
- 32, 32': Shaft
- 33: Pillar base
- 34: Cabinet
- 35, 35': Pillar
- 36, 36': Shaft
- 37: Pillar base
- 38: Cross beam
- 39: Cross beam
- 40, 40': Guide hose
- 41, 41': Guide hose
- 50: Support system
- 51, 51': Pillar
- 52, 52': Pillar
- 53: Pillar base
- 54: Pillar base
- 55, 55': Shaft
- 56, 56': Shaft
- 57: Cross beam
- 58: Cross beam
- 59: Cabinet
- 60: Guide hose
- 61: Guide hose
- 62: Port
- 63: Profile
- 64: Cover
- 65: Horizontal hole
- 66: Mounting block
- 67: Vertical hole
- 68: Mounting block
- 69: Hole
- 70: Pillar
- 71: Pillar
- 72: Guide sleeve
- 73: Guide sleeve
- 74: Guide shaft
- 75: Guide shaft
- 76: Upper pulley
- 77: Upper pulley
- 78: Lower pulley
- 79: Lower pulley
- 80: Cable
- 81: First end
- 82: Second end
- 83: Drum
- 90: Rail
- 91: Support
- 92, 92': Pillar
- 93: Cross beam
- 94, 94': Foot plate
- 95: Pillar
- 96, 96': Door wing
- 97: Passage
- 98: Cabinet
- 100: Wheel
- 101: Lever
- 102: Wheel
- 103: Bar
- 104: Trolley

## Claims

1. Medical equipment support system, comprising a support structure extending in a substantially horizontal direction for supporting at least one medical device, at least two supports arranged at opposing ends of the support structure for supporting the support structure in an overhead position, wherein the at least two supports are pillars (3, 3', 4, 4', 31, 31', 35, 35', 51, 51', 52, 52', 70, 71, 92, 92', 95) extending in a substantially vertical direction, being adapted for standing on a floor (6) of a medical room, wherein the support structure is at least one beam (7, 7') and/or platform supporting at least one holder (10) to which the at least one medical device can be mounted, wherein the at least one beam (7, 7') and/or platform comprises a rail (90) and the at least one holder (10) is movable along the rail (90), **characterized in that** the holder (10) is automatically movable into at least one preference position according to a predetermined medical scheme and that the support structure is connectable to the support structure of a further medical equipment support system for increasing a range of positions at which medical equipment can be suspended.

2. Medical equipment support system according to claim 1, **characterized in that** the pillars (3, 3', 4, 4', 31, 31', 35, 35', 51, 51', 52, 52', 70, 71, 92, 92', 95) are equipped with wheels (102) and/or gliding elements for moving the pillars (3, 3', 4, 4', 31, 31', 35, 35', 51, 51', 52, 52', 70, 71, 92, 92', 95) on the floor (6).

3. Medical equipment support system according to any one of the preceding claims, **characterized in that** the support structure is removably fixed to the pillars (3, 3', 4, 4', 31, 31', 35, 35', 51, 51', 52, 52', 70, 71, 92, 92', 95).

4. Medical equipment support system according to any one of the preceding claims, **characterized in that** the pillars (31, 31', 35, 35', 51, 51', 52, 52', 70, 71) are adapted for moving the support structure in the substantially vertical direction.

5. Medical equipment support system according to any one of the preceding claims, **characterized in that** the pillars (3, 3', 4, 4', 31, 31', 35, 35', 51, 51', 52, 52', 70, 71, 95) and/or the support structure are configured for accommodating cables and/or fluid lines.

6. Medical equipment support system according to any one of the preceding claims, **characterized in that** at least one of the pillars (3, 3', 4, 4', 31, 31', 35, 35', 51, 51', 52, 52', 70, 71, 95) comprises a cabinet (14, 14', 34, 59, 98) for placing medical equipment.

7. Medical equipment support system according to any one of the preceding claims, **characterized in that** the support structure is supported by at least two pillars (3, 3', 4, 4', 70, 71, 92, 92', 95), each having a strut or a foot plate (15, 16, 94, 94') for stable standing on the floor (6).

8. Medical equipment support system according to any one of the preceding claims, **characterized in that** the support structure is one beam (7) or a multiplicity of parallel beams (7, 7') and that at least one end of the beam (7) or beams (7, 7') is supported by at least two pillars (3, 3', 4, 4', 31, 31', 35, 35', 51, 51', 52, 52', 70, 71, 92, 92').

9. Medical equipment support system according to claim 8, **characterized in that** the at least two pillars (31, 31', 35, 35', 51, 51', 52, 52', 70, 71, 92, 92') are rigidly connected to form a pillar unit.

10. Medical equipment support system according to any one of the preceding claims, **characterized in that** the holder (10) is manually movable into the at least one preference position, the preference position being indicated by a stop.

## Patentansprüche

1. Tragesystem für medizinische Ausrüstung, umfassend eine Tragestruktur, die sich in einer im Wesentlichen horizontalen Richtung erstreckt, um wenigstens eine medizinische Vorrichtung zu tragen, wobei wenigstens zwei Stützen an gegenüberliegenden Enden der Tragestruktur angeordnet sind, um die Tragestruktur in einer Überkopfposition zu halten, wobei die wenigstens zwei Stützen Stützpfeiler (3, 3', 4, 4', 31, 31', 35, 35', 51, 51', 52, 52', 70, 71, 92, 92', 95) sind, die sich in einer im Wesentlichen vertikalen Richtung erstrecken und die dafür ausgelegt sind, auf einem Boden (6) eines Behandlungsraumes zu stehen, wobei die Tragestruktur wenigstens einen Träger (7, 7') und/oder wenigstens eine Plattform umfasst, die wenigstens eine Halterung (10) tragen, an der die wenigstens eine medizinische Vorrichtung montiert werden kann, wobei der wenigstens eine Träger (7, 7') und/oder die wenigstens eine Plattform eine Schiene (90) umfasst und die wenigstens eine Halterung (10) entlang der Schiene (90) beweglich ist, **dadurch gekennzeichnet, dass** die Halterung (10) automatisch in wenigstens eine Vorzugsposition gemäß einem vorbestimmten medizinischen Plan beweglich ist und dass die Tragestruktur mit der Tragestruktur eines weiteren Tragesystems für medizinische Ausrüstung verbunden werden kann, um einen Bereich von Positionen zu erweitern, in denen die medizinische Ausrüstung aufgehängt werden kann.

2. Tragesystem für medizinische Ausrüstung gemäß Anspruch 1, **dadurch gekennzeichnet, dass** die Stützpfeiler (3, 3', 4, 4', 31, 31', 35, 35', 51, 51', 52, 52', 70, 71, 92, 92', 95) mit Rädern (102) und/oder Gleitelementen zum Verschieben der Stützpfeiler (3, 3', 4, 4', 31, 31', 35, 35', 51, 51', 52, 52', 70, 71, 92, 92', 95) auf dem Boden (6) ausgerüstet sind.

3. Tragesystem für medizinische Ausrüstung gemäß einem der vorstehenden Ansprüche, **dadurch gekennzeichnet, dass** die Tragestruktur abnehmbar an den Stützpfeilern (3, 3', 4, 4', 31, 31', 35, 35', 51, 51', 52, 52', 70, 71, 92, 92', 95) befestigt ist.

4. Tragesystem für medizinische Ausrüstung gemäß einem der vorstehenden Ansprüche, **dadurch gekennzeichnet, dass** die Stützpfeiler (31, 31', 35, 35', 51, 51', 52, 52', 70, 71) dafür ausgelegt sind, die Tragestruktur in der im Wesentlichen vertikalen Richtung zu bewegen.

5. Tragesystem für medizinische Ausrüstung gemäß einem der vorstehenden Ansprüche, **dadurch gekennzeichnet, dass** die Stützpfeiler (3, 3', 4, 4', 31, 31', 35, 35', 51, 51', 52, 52', 70, 71, 95) und/oder die Tragestruktur dafür ausgelegt sind, Kabel und/oder Fluidleitungen aufzunehmen.

6. Tragesystem für medizinische Ausrüstung gemäß einem der vorstehenden Ansprüche, **dadurch gekennzeichnet, dass** wenigstens einer der Stützpfeiler (3, 3', 4, 4', 31, 31', 35, 35', 51, 51', 52, 52', 70, 71, 95) einen Schrank (14, 14', 34, 59, 98) umfasst, um die medizinische Ausrüstung unterzubringen.

7. Tragesystem für medizinische Ausrüstung gemäß einem der vorstehenden Ansprüche, **dadurch gekennzeichnet, dass** die Tragestruktur von wenigstens zwei Stützpfeilern (3, 3', 4, 4', 70, 71, 92, 92', 95) getragen wird, die jeweils eine Verstrebung oder eine Bodenplatte (15, 16, 94, 94') aufweisen, für einen stabilen Stand auf dem Boden (6).

8. Tragesystem für medizinische Ausrüstung gemäß einem der vorstehenden Ansprüche, **dadurch gekennzeichnet, dass** die Tragestruktur ein Einzelträger (7) oder eine Mehrzahl paralleler Träger (7, 7') ist und dass wenigstens ein Ende des Trägers (7) oder der Träger (7, 7') von wenigstens zwei Stützpfeilern (3, 3', 4, 4', 31, 31', 35, 35', 51, 51', 52, 52', 70, 71, 92, 92') getragen wird.

9. Tragesystem für medizinische Ausrüstung gemäß Anspruch 8, **dadurch gekennzeichnet, dass** die wenigstens zwei Stützpfeiler (31, 31', 35, 35', 51, 51', 52, 52', 70, 71, 92, 92') starr verbunden sind, um eine Stützpfeilereinheit zu bilden.

10. Tragesystem für medizinische Ausrüstung gemäß einem der vorstehenden Ansprüche, **dadurch gekennzeichnet, dass** die Halterung (10) von Hand in die wenigstens eine Vorzugsposition beweglich ist, wobei die Vorzugsposition durch einen Anschlag angezeigt wird.

## Revendications

1. Système de support d'équipement médical, comprenant une structure de support s'étendant dans une direction sensiblement horizontale pour supporter au moins un dispositif médical, au moins deux supports disposés à des extrémités opposées de la structure de support pour supporter la structure de support dans une position suspendue, les au moins deux supports étant des piliers (3, 3', 4, 4', 31, 31', 35, 35', 51, 51', 52, 52', 70, 71, 92, 92', 95) s'étendant dans une direction sensiblement verticale, prévus pour reposer sur un sol (6) d'une salle médicale, la structure de support étant au moins une poutre (7, 7') et/ou plate-forme supportant au moins un dispositif de retenue (10) sur lequel l'au moins un dispositif médical peut être monté, l'au moins une poutre (7, 7') et/ou plate-forme comprenant un rail (90) et l'au moins un dispositif de retenue (10) pouvant être déplacé le long du rail (90), **caractérisé en ce que** le dispositif de retenue (10) peut être déplacé automatiquement dans au moins une position préférentielle suivant un schéma médical prédéterminé et **en ce que** la structure de support peut être connectée à la structure de support d'un système de support d'équipement médical supplémentaire pour augmenter la plage de positions auxquelles un équipement médical peut être suspendu.

2. Système de support d'équipement médical selon la revendication 1, **caractérisé en ce que** les piliers (3, 3', 4, 4', 31, 31', 35, 35', 51, 51', 52, 52', 70, 71, 92, 92', 95) sont équipés de roues (102) et/ou d'éléments glissants pour déplacer les piliers (3, 3', 4, 4', 31, 31', 35, 35', 51, 51', 52, 52', 70, 71, 92, 92', 95) sur le sol (6).

3. Système de support d'équipement médical selon l'une quelconque des revendications précédentes, **caractérisé en ce que** la structure de support est fixée de manière amovible aux piliers (3, 3', 4, 4', 31, 31', 35, 35', 51, 51', 52, 52', 70, 71, 92, 92', 95).

4. Système de support d'équipement médical selon l'une quelconque des revendications précédentes, **caractérisé en ce que** les piliers (31, 31', 35, 35', 51, 51', 52, 52', 70, 71) sont prévus pour déplacer la structure de support dans la direction sensiblement verticale.

5. Système de support d'équipement médical selon l'une quelconque des revendications précédentes, **caractérisé en ce que** les piliers (3, 3', 4, 4', 31, 31', 35, 35', 51, 51', 52, 52', 70, 71, 95) et/ou la structure de support sont configurés pour recevoir des câbles et/ou des conduites de fluide.

6. Système de support d'équipement médical selon l'une quelconque des revendications précédentes, **caractérisé en ce qu'**au moins l'un des piliers (3, 3', 4, 4', 31, 31', 35, 35', 51, 51', 52, 52', 70, 71, 95) comprend une armoire (14, 14', 34, 59, 98) pour placer un équipement médical.

7. Système de support d'équipement médical selon l'une quelconque des revendications précédentes, **caractérisé en ce que** la structure de support est supportée par au moins deux piliers (3, 3', 4, 4', 70, 71, 92, 92', 95) ayant chacun une entretoise ou une plaque de pied (15, 16, 94, 94') pour reposer de manière stable sur le sol (6).

8. Système de support d'équipement médical selon l'une quelconque des revendications précédentes, **caractérisé en ce que** la structure de support est une poutre (7) ou une pluralité de poutres parallèles (7, 7') et **en ce qu'**au moins une extrémité de la poutre (7) ou des poutres (7, 7') est supportée par au moins deux piliers (3, 3', 4, 4', 31, 31', 35, 35', 51, 51', 52, 52', 70, 71, 92, 92').

9. Système de support d'équipement médical selon la revendication 8, **caractérisé en ce que** les au moins deux piliers (31, 31', 35, 35', 51, 51', 52, 52', 70, 71, 92, 92') sont connectés rigidement pour former une unité de pilier.

10. Système de support d'équipement médical selon l'une quelconque des revendications précédentes, **caractérisé en ce que** le dispositif de retenue (10) peut être déplacé manuellement dans l'au moins une position préférentielle, la position préférentielle étant indiquée par une butée.
